# EUROPEAN PATENT APPLICATION

(11) **EP 4 349 989 A1**
(43) Date of publication of application: **10.04.2024**
(21) Application number: 22815324.3
(22) Date of filing: 01.06.2022
(51) Int. Cl.: C12N 15/63, C12N 9/10, C12P 19/00

(54) **GLYCOSYLTRANSFERASE AND APPLICATION THEREOF**

(30) Priority: 01.06.2021 CN 202110610098; 24.12.2021 CN 202111602506
(71) Applicant: Abiochem Biotechnology Co., Ltd., Shanghai 200241 (CN)
(72) Inventor: WU, Yan, Shanghai 200241 (CN); TIAN, Zhenhua, Shanghai 200241 (CN); WANG, Shu, Shanghai 200241 (CN); ZHENG, Xiaofu, Shanghai 200241 (CN)
(74) Representative: Secerna LLP
(86) International application number: PCT/CN2022/096683
(87) International publication number: WO 2022/253282

(57) **Abstract**

A glycosyltransferase and an application thereof, the amino acid sequence of the glycosyltransferase being shown as in SEQ ID NO: 112 or an amino acid sequence having at least 99% sequence identity to SEQ ID NO: 112. The glycosyltransferase of the present invention has high enzyme activity and good stability and, when used for the preparation of steviol glycosides, has a significant improvement in catalytic activity and significantly improved conversion rate compared to the parent glycosyltransferase, solving the problem of the high price of the glycosyl donor UDPG (and/or ADPG), and thereby reducing the reaction costs and facilitating industrial production.

## Description

The present application claims the priorities of Chinese patent application 2021106100982 filed on June 1, 2021, and Chinese patent application 2021116025066 filed on December 24, 2021. The contents of the above Chinese patent applications are incorporated herein by reference in its entirety.

### TECHNICAL FIELD

The present invention relates to a glycosyltransferase and an application thereof in glycosylation reaction of steviol glycosides.

### BACKGROUND

Steviol glycosides are natural sweeteners extracted from the leaves of the Asteraceae herb *stevia rebaudiana.* They are a mixture of various glycosides, and there are significant differences in taste quality among different steviol glycosides. Steviol glycosides possess several advantages: they are pure natural (derived from the pure natural plant *stevia rebaudiana*), highly sweet (250-450 times sweeter than sucrose), low in calories (only 1/300th of white sugar), economical to use (only one-third of the cost of sucrose), stable (resistant to heat, acids, and alkalis, and less prone to decomposition), and highly safe (non-toxic side effects). Additionally, they have potential therapeutic effects of anti-hyperglycemia, anti-hypertension, anti-inflammation, anti-tumor, and anti-diarrhea.

The structural formula of steviol glycosides (steviol glycoside compounds) is as follows:

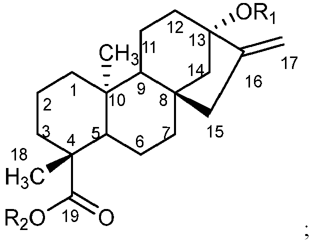

| No. | Compound | R₁ | R₂ |
|---|---|---|---|
| 1 | Steviol | H | H |
| 2 | Steviolmonoside | H | β-Glc |
| 3 | Steviolbioside | H | β-Glc-β-Glc(2-1) |
| 4 | Rubusoside | β-Glc | β-Glc |
| 5 | Stevioside (STV) | β-Glc | β-Glc-β-Glc(2-1) |
| 6 | Rebaudioside A (RA) | β-Glc | β-Glc-β-Glc(2-1)- β-Glc(3-1) |
| 7 | Rebaudioside B (RB) | H | P-Glc-P-Glc(2-1)- β-Glc(3-1) |
| 8 | Rebaudioside C (RC) | β-Glc | β-Glc-α-Rha(2-1)- β-Glc(3-1) |
| 9 | Rebaudioside D (RD) | β-Glc-β-Glc(2-1) | β-Glc-β-Glc(2-1)- β-Glc(3-1) |
| 10 | Rebaudioside E (RE) | β-Glc-β-Glc(2-1) | β-Glc-β-Glc(2-1) |
| 11 | Rebaudioside F (RF) | β-Glc | β-Glc-α-Xly(2-1)- β-Glc(3-1) |
| 12 | Rebaudioside M (RM) | β-Glc-β**-**Glc(2-1)-β-Glc(3-1) | β-Glc-β-Glc(2-1)- β-Glc(3-1) |
| 13 | Dulcoside A | β-Glc | β-Glc-α-Rha(2-1) |
| 14 | Rebaudioside I (RI) | β-Glc-β-Glc(3-1) | β-Glc-β-Glc(2-1)- β-Glc(3-1) |

The aforementioned steviol glycoside compounds share a common aglycone: steviol. Their distinction lies in the number and type of glycosyl groups connected at the C-13 and C-19 positions. They primarily include stevioside, rebaudioside A (Reb A), rebaudioside B, rebaudioside C, rebaudioside D, rebaudioside E, rebaudioside I, dulcoside, steviolbioside, and other glycosides. The leaves of *stevia rebaudiana* can accumulate up to 10-20% (based on dry weight) of steviol glycosides. The primary glycosides found in the leaves of *stevia rebaudiana* plant are rebaudioside A (2-10%), stevioside (2-10%), and rebaudioside C (1-2%). Other glycosides such as rebaudioside B, D, E, F, and I, along with steviolbioside and rubusoside, are found in significantly lower levels in the leaves (approximately 0-0.2%). Among these, rebaudioside A has the highest sweetness, which closely resembles that of sucrose. It is a new type of natural sweetener that is high in sweetness, low in calories, easy to dissolve, heat-resistant, and stable. The content or purity of rebaudioside A is also a primary indicator of the quality of stevioside. Additionally, rebaudioside A serves as a crucial intermediate in the synthesis of other steviol glycoside compounds, such as rebaudioside D (RebD, RD), rebaudioside E (RebE, RE), rebaudioside M (RebM, RM), rebaudioside I (RebI, RI), etc.

Although steviol glycosides are high-intensity sweeteners, their application in fields with high taste requirements such as food and beverages, is seriously limited by their lingering bitter aftertaste. The underlying cause of this aftertaste is the intrinsic molecular structure of steviol glycosides. The more glycosyl groups connected to the R₁ and R₂ groups, the better is the taste. Typically, it is found that stevioside is 110-270 times sweeter than sucrose, and rebaudioside A is 150 to 320 times sweeter. However, even in highly purified forms, steviol glycosides still possess undesirable taste attributes, such as bitterness, sweet aftertaste, and licorice flavor, etc.

Rebaudioside D is one of the steviol glycosides with the most potential for application. Compared with other steviol glycosides, its sweetness is high, about 300 to 350 times that of sucrose. It has a pure sweetness and a taste closer to sucrose, without any bitterness or licorice-like off-flavor, and good stability, making it an ideal natural high-intensity sweetener. However, the content of rebaudioside D in the leaves of *stevia rebaudiana* is very low (less than 5%), making its production through extraction methods requires a large amount of *stevia rebaudiana* raw material. Additionally, the process of enriching rebaudioside D is complicated, it needs column passes, desalting, decolorization, and recrystallization for several times after extraction. This process generates a considerable amount of wastewater, leading to high production costs, which is not suitable for large-scale industrial production.

The current method of bio-enzymatic synthesis of rebaudioside D requires the addition of costly UDP-glucose as one of the substrates. The process involves the action of UDP-glucosyltransferase (UGT) and using stevioside or rebaudioside A as the substrate to catalyze the formation of rebaudioside D. However, due to the extremely high selling price of UDP-glucose, the feasibility of industrial preparation of rebaudioside D is almost completely limited, resulting in poor economic efficiency and lack of market competitiveness.

Rebaudioside M (RebM) has superior taste properties, but its content in the dry weight of leaves is less than 0.1%, leading to high isolation costs and high price. The biocatalytic method to obtain high concentrations of rebaudioside M has attracted the attention of scholars. Currently, it is reported that the recombinant enzymes derived from *stevia rebaudiana* can catalyze rebaudioside D to produce rebaudioside M, but the yield is relatively low. Using rebaudioside D as the substrate, rebaudioside M can be yielded by the microbial enzyme-producing catalysis. Compared with conventional extraction methods, this method not only improves the production process, but also reduces environmental pollution and increases the yield of the desired product, rebaudioside M. The current biological enzyme catalysis method mainly has the following problems: (1) The cost of using biological enzymes to catalyze the production of rebaudioside M from rebaudioside D is relatively high, and the enzyme-catalyzed yield needs to be further optimized; (2) The glycosyltransferase used for catalysis is difficult to be separated from the product and recycled, and is prone to deactivation; (3) In natural plants, the content of rebaudioside A is quite high, whereas that of rebaudioside D is very low. It is also a difficult problem to be solved urgently to directly convert rebaudioside A into rebaudioside D at low cost.

Glucosyltransferase is an enzyme that only transfers glucosyl groups in enzyme reactions. Its mechanism of action involves catalyzing the transfer of the glucose residues from a glycosyl donor to a glycosyl acceptor molecule, thereby regulating the activity of the acceptor molecule.

"Nucleoside" refers to glycosylamines that contain a nucleobase (i.e., a nitrogenous base) and a 5-carbon sugar (such as ribose or deoxyribose). Non-limiting examples of nucleosides include cytidine, uridine, adenosine, guanosine, thymidine, and inosine.

"Nucleoside diphosphate" refers to a glycosylamine containing a nucleobase (i.e., a nitrogenous base), a 5-carbon sugar (such as ribose or deoxyribose), and a diphosphate (i.e., pyrophosphate) moiety. "Nucleoside diphosphate" can be abbreviated as "NDP". Non-limiting examples of nucleoside diphosphate include cytidine diphosphate (CDP), uridine diphosphate (UDP), adenosine diphosphate (ADP), guanosine diphosphate (GDP), thymidine diphosphate (TDP), and inosine diphosphate (IDP).

UDP-glucosyltransferase is a type of glucosyltransferase that uses UDP-glucose as the glycosyl donor. It is found in almost all organisms. UDP-glucose is the abbreviation of uridine diphosphate glucose, also abbreviated as UDP-Glc or UDPG, is a vitamin composed of uridine diphosphate and glucose. It can be considered as "active glucose" and is widely distributed in the cells of plants, animals, and microorganisms. It acts as a glucosyl donor in the synthesis of sucrose, starch, glycogen, and other oligosaccharides and polysaccharides, making it the most common type of glycosyl donors. In addition to UDP-glucose, other glycosyl donor compounds such as ADP-glucose, TDP-glucose, dTDP-glucose (deoxythymidine diphosphate glucose), CDP-glucose, IDP-glucose, or GDP-glucose are also commonly used. These glycosyl donors are expensive, which is not conducive to industrial-scale production.

Sucrose synthase (SUS, also abbreviated as SuSy/SS, EC2.4.1.13) which reversibly catalyzes the chemical reaction of NDP-glucose + D-fructose to NDP and sucrose. It belongs to the glycosyltransferase-4 subfamily and was first identified in the embryo of wheat (*Triticum aestivum*)*.* Each protein exists in a tetrameric form. The molecular weight of each subunit is around 90 kDa. The size of the gene is typically 5.9 kb, and the size of the cDNA is about 2.7 kb. The encoded amino acid sequence is about 800 residues in full length (Ross and Davies 1992). Sucrose synthase shows varying affinities for different NDPs, with the order of affinity being UDP > ADP > dTDP > CDP > GDP.

Ohta et al. (J. Appl. Glycosci., 57, 199~209 (2010)) first reported the isolation of rebaudioside I (Reb I, RI) from *stevia rebaudiana.* Indra Prakash et al. (Molecules 2014, 19, 17345-17355) first reported a biosynthetic method for preparing RI by using the mutant UGT76G1-R11-F12 of glucosyltransferase UGT76G1 from RA (Rebaudioside A) as raw material. This method used UDP-glucose as the glycosyl donor. The reaction was carried out in a shake flask at 30°C under conditions of pH 7.5 and MgCl₂, resulting in a 22.5% yield of RI. In the patent application CN106795523A, in which Indra Prakash is listed as the inventor, the GenBank accession number AAR06912.1 for the glucosyltransferase UGT76G1 is disclosed, along with the DNA sequence of this enzyme. CN110914445A screened mutants of glucosyltransferase UGT76G1 that catalyze the preparation of RI from RA as raw material. The screening conditions were pH 7.0, MgCh 10.3 mM, and temperature of 35°C. CN106795523A reported the sweetness data of RI, and compositions containing RI. The above-mentioned prior art only discloses glucosyltransferases or mutants thereof with the activity of catalyzing RA to synthesize RI, but these enzymes have low activity and are not suitable for industrial-scale production.

Nowadays, with the widespread application of the natural sweetener stevioside and the continuous development of biocatalysis technology, UDP-glucosyltransferase is increasingly used in the field of biocatalytic preparation of steviol glycosides. There are many types of UDP-glucosyltransferases. Currently, the enzymes used in the field of bio-enzymatic preparation of steviol glycosides are mostly wild-type enzymes derived from plant cells. These wild-type enzymes often have drawbacks such as low enzyme activity and poor stability, leading to high costs for industrial-scale production of steviol glycosides. Therefore, it is necessary to modify UDP-glucosyltransferases to obtain modified enzymes with higher enzymatic activity and better stability, in order to better serve industrial-scale production.

### CONTENT OF THE PRESENT INVENTION

The technical problem to be solved by the present disclosure is that the existing UDP-glucosyltransferases has low enzymatic activity and poor stability when applied to the biocatalytic preparation of steviol glycosides, resulting in a low conversion rate when used to catalyze steviol glycosides. Therefore, the present disclosure provides a glycosyltransferase (i.e., UDP-glucosyltransferase) and an application thereof in the preparation of steviol glycosides. The glycosyltransferase (GT) of the present disclosure exhibits high enzymatic activity and good stability; wherein, sucrose and UDP (and/or ADP) generate UDPG (and/or ADPG) under the catalysis of SUS, thereby achieving the regeneration of UDPG (and/or ADPG) and solving the problem of the expensive price of the glycosyl donor UDPG (and/or ADPG). When it is used to prepare steviol glycosides (such as rebaudioside A, rebaudioside D, rebaudioside M, or rebaudioside I), the catalytic activity and the conversion rate are significantly improved compared with those of the glycosyltransferase parent, thereby reducing the cost of the reaction and providing more options for optimizing the process conditions for realizing large-scale industrial production, which is conducive to industrial-scale production.

In order to solve the above technical problems, the present disclosure provides a glycosyltransferase, wherein an amino acid sequence of the glycosyltransferase is shown as SEQ ID NO: 112, or an amino acid sequence having at least 99% sequence identity with SEQ ID NO: 112.

Preferably, the amino acid sequence having at least 99% sequence identity with SEQ ID NO: 112 contains differences in amino acid residues selected from one or more of the following residue positions compared to SEQ ID NO: 112:
V14I;
E99L;
T254G;
L257A;
Q451E;
Q265E;
P271A;
R333K;
R12Q;
A118S;
E418D;
S455R.

More preferably, on the basis of the above, the amino acid sequence of the glycosyltransferase can further contain differences in amino acid residues selected from the following residue position compared to SEQ ID NO: 112: K347P.

In one preferred embodiment, the amino acid sequence of the glycosyltransferase, contains differences in amino acid residues selected from one of the following residue positions compared to SEQ ID NO: 112: V14I, E99L, T254G, L257A, Q451E, Q265E, P271A, R333K, R12Q, A118S, E418D, or S455R.

In one preferred embodiment, the amino acid sequence of the glycosyltransferase contains differences in amino acid residues selected from the following two residue positions compared to SEQ ID NO: 112: Q265E and P271A.

In one preferred embodiment, the amino acid sequence of the glycosyltransferase contains differences in amino acid residues selected from the following two residue positions compared to SEQ ID NO: 112: R333K and K347P.

In one preferred embodiment, a nucleotide sequence encoding the glycosyltransferase can be selected from the following sequences: SEQ ID NO: 41, SEQ ID NO: 92, SEQ ID NO: 93, SEQ ID NO: 94, SEQ ID NO: 95, SEQ ID NO: 97, SEQ ID NO: 98, SEQ ID NO: 99, SEQ ID NO: 100, SEQ ID NO: 101, SEQ ID NO: 102, SEQ ID NO: 103, SEQ ID NO: 104, SEQ ID NO: 107, and SEQ ID NO: 108.

In some embodiments, the amino acid sequence of the glycosyltransferase may additionally have differences in 1 to 2, 1 to 3, 1 to 4, 1 to 5, 1 to 6, 1 to 7, 1 to 8, 1 to 9, 1 to 10, 1 to 11, 1 to 12, 1 to 13, 1 to 14, 1 to 15, 1 to 16, 1 to 17, 1 to 18, 1 to 19, or 1 to 20 amino acid residues at positions other than the specific positions described above; these residue differences include substitutions with conservative amino acid residues. Typically, these substitutions do not affect the enzymatic activity of the glycosyltransferase.

In order to solve the above technical problems, the present disclosure provides an isolated nucleic acid, which encodes the glycosyltransferase as described above.

Preferably, a nucleotide sequence of the nucleic acid can be selected from the following sequences: SEQ ID NO: 41, SEQ ID NO: 92, SEQ ID NO: 93, SEQ ID NO: 94, SEQ ID NO: 95, SEQ ID NO: 97, SEQ ID NO: 98, SEQ ID NO: 99, SEQ ID NO: 100, SEQ ID NO: 101, SEQ ID NO: 102, SEQ ID NO: 103, SEQ ID NO: 104, SEQ ID NO: 107, and SEQ ID NO: 108.

In order to solve the above technical problems, the present disclosure provides a recombinant expression vector comprising the nucleic acid as described above.

In order to solve the above technical problems, the present disclosure provides a transformant, which is a host cell comprising the nucleic acid as described above or the recombinant expression vector as described above.

The host cell can be conventional in the field, preferably *Escherichia coli.*

In order to solve the above technical problems, the present disclosure provides a method for preparing the glycosyltransferase as described above, which comprises culturing the transformant as described above under conditions suitable for expressing the glycosyltransferase.

After the transformant expresses the glycosyltransferase, conventional techniques in the field can be used for extraction, for example, a crude enzyme solution can be prepared. After the crude enzyme solution is prepared, it can be subjected to conventional concentration and replacement. The crude enzyme solution can also be further purified through one or more purification steps such as ion exchange chromatography, affinity chromatography, hydrophobic chromatography, and molecular sieve chromatography, to purify the glycosyltransferase. In one preferred embodiment, the following steps can be employed: (1) inoculating the transformant containing the glycosyltransferase into a culture medium containing antibiotics, such as LB medium, and performing shaking culture to obtain seed solution; (2) transferring the seed solution obtained in step (1) to a culture medium containing antibiotics, such as TB medium, for shaking cultivation; (3) adding IPTG to the culture medium obtained in step (2) to induce expression overnight, and collecting bacterial cells after centrifugation; (4) washing and resuspending the bacterial cells collected in step (3), crushing and centrifuging to obtain the crude enzyme solution containing the glycosyltransferase.

In order to solve the above technical problems, the present disclosure provides a composition comprising the glycosyltransferase as described above. The composition can exist, for example, in the form of an enzyme preparation.

In order to solve the above technical problems, the present disclosure provides a method for glycosylation of a substrate, which includes providing at least one substrate and the glycosyltransferase as described above, and contacting the substrate with the glycosyltransferase under conditions that cause the substrate to be glycosylated to produce at least one glycosylation product; the substrate preferably includes at least one steviol glycoside, such as stevioside, rebaudioside A, or rebaudioside D, etc.

In order to solve the above technical problems, the present disclosure provides a preparation method for rebaudioside A. The preparation method includes the following steps: in the presence of the glycosyltransferase as described above, a reaction between stevioside and a glycosyl donor is carried out (for example, under conditions that cause the stevioside to be glycosylated to produce rebaudioside A), thereby obtaining rebaudioside A.

Preferably, the glycosyltransferase exists in the form of a bacterial sludge.

Preferably, a concentration of the stevioside is 1 to 150 g/L, more preferably 100 g/L.

Preferably, a molar ratio of the glycosyl donor to the stevioside is 1:1 to 5:1, for example, 2.5:1.

Preferably, the glycosyl donor is UDP-glucose. In one preferred embodiment, the glycosyl donor used is preferably prepared by sucrose and UDP in the presence of sucrose synthase, and a concentration of the sucrose is preferably 100 to 300 g/L, for example, 200 g/L, and a concentration of the UDP is preferably 0.05 to 0.2 g/L, for example, 0.1 g/L. Synthases, such as sucrose synthase or trehalose synthase, typically act in opposite directions to form nucleoside diphosphate glucose compounds from nucleoside diphosphates and glucose donors (e.g., sucrose, trehalose, or starch). In the present disclosure, the sucrose synthase (abbreviated as SuSy or SUS) catalyzes a reaction of reversible glucosyl transfer, and the sucrose synthase catalyzes sucrose and UDP to produce UDP-glucose, effectively providing a UDP-sugar donor for the UDP-glycosyltransferase. In a reaction system constructed by sucrose synthase and UDP-glycosyltransferase, a UDP cycle is generated to realize the regeneration of UDP-glucose, which avoids the direct use of expensive UDP-glucose and reduces production costs.

Preferably, a reaction solvent used in the reaction has a pH between 5 and 8, more preferably 6.

Preferably, a rotation speed during the reaction is 500 to 1000 rpm, more preferably 600 rpm.

Preferably, a temperature of the reaction system for the reaction is 20 to 90°C, more preferably 60°C.

In order to solve the above technical problems, the present disclosure provides a preparation method for rebaudioside D or rebaudioside M, which includes the step of preparing rebaudioside A according to the preparation method as described above.

In one preferred embodiment, the method includes providing a stevioside substrate, a glycosyl donor, and the glycosyltransferase as previously described, and reacting the stevioside substrate, the glycosyl donor, and the glycosyltransferase as previously described under conditions that enable the production of rebaudioside D or rebaudioside M.

In order to solve the above technical problem, the present disclosure provides a preparation method for rebaudioside I, wherein rebaudioside A and a glycosyl donor are reacted in the presence of the glycosyltransferase as described above.

In the preparation method, the glycosyltransferase exists in the form of glycosyltransferase cells, crude enzyme solution, pure enzyme, pure enzyme solution, or immobilized enzyme.

In the preparation method, the glycosyl donor is UDP-glucose and/or ADP-glucose.

In the preparation method, a concentration of the rebaudioside Ais preferably 1 to 150 g/L, for example, 100 g/L, 50 g/L, 80 g/L, 120 g/L.

In the preparation method, a mass ratio of the glycosyltransferase cells to rebaudioside A is preferably 1:(3-10), for example, 3:20, 1:5, 1:8.

In the preparation method, a reaction solvent used in the reaction has a pH preferably between 5 and 8, more preferably between 5.5 and 6.

In the preparation method, a temperature of a reaction system for the reaction is preferably 20 to 90°C, for example, 60°C, 30°C, 50°C, 70°C.

In the preparation method, a reaction time for the reaction is preferably 5 to 30 h, for example, 24 h, 10 h, 15 h, 20 h, 28 h.

In the preparation method, the glycosyl donor is preferably prepared by UDP and/or ADP in the presence of sucrose and sucrose synthase.

A concentration of the sucrose is preferably 100 to 300 g/L, for example, 200 g/L, 150 g/L, 250 g/L.

A concentration of the UDP or the ADP is preferably 0.05 to 0.2 g/L, for example, 0.1 g/L.

In the preparation method, the pH is preferably controlled by a buffer solution, and the buffer solution is, for example, a phosphate buffer solution.

In the preparation method, a rotation speed of the reaction is preferably 500 to 1000 rpm, for example, 600 rpm, 800 rpm.

In order to solve the above technical problems, the present disclosure provides a use of the glycosyltransferase as described above, in the preparation of steviol glycosides; the steviol glycosides are preferably rebaudioside A, rebaudioside D, rebaudioside M, or rebaudioside I.

In order to solve the above technical problems, the present disclosure provides a catalytic enzyme composition comprising a glycosyltransferase and a sucrose synthase, and the glycosyltransferase is as described above, and the sequence of the sucrose synthase is as shown in SEQ ID NO: 32.

In the catalytic enzyme composition, a mass ratio of the glycosyltransferase to the sucrose synthase is preferably (2-10):1, for example, 5:1, 3:1, or 8:1.

In order to solve the above technical problems, the present disclosure provides an application of the catalytic enzyme composition as described above in the preparation of rebaudioside A, rebaudioside D, rebaudioside M, or rebaudioside I.

In the present disclosure, steviol glycosides are detailed in terms of their structural formulas and the types of compounds included therein. For details please refer to the background section of the present disclosure.

The positive progressive effects of the present disclosure are as follows:

The glycosyltransferase of the present disclosure exhibits high enzymatic activity and good stability. When it is used to prepare steviol glycosides (such as rebaudioside A, rebaudioside D, rebaudioside M, or rebaudioside I), the catalytic activity and conversion rate are significantly improved compared with those of the glycosyltransferase parent. It solves the problem of the expensive price of the glycosyl donor UDPG (and/or ADPG), thereby reducing the cost of the reaction and providing more options for optimizing the process conditions for realizing large-scale industrial production, and being beneficial to industrial-scale production.

### BRIEF DESCRIPTION OF THE DRAWINGS

Figure 1 presents a schematic diagram of the route for preparing rebaudioside A, rebaudioside D, and rebaudioside M from stevioside in examples 1-7 of the present disclosure.
Figure 2 presents the enzyme-catalyzed reaction system in examples 1-7 of the present disclosure for the synthesis of rebaudioside A, which achieves the cycling of UDPG in the biocatalytic reaction.
Figure 3 presents a chromatogram of the substrate stevioside used in the second round of screening in examples 1-7, with a retention time of 12.76 min.
Figure 4 presents a chromatogram of the reference substance of the product rebaudioside A in examples 1-7, with a retention time of 12.38 min.
Figure 5 is an HPLC chromatogram of the activity of RA catalytic synthesized by the re-screened Enz. 11 in Table 10.
Figure 6 is an HPLC chromatogram of the activity of RA catalytic synthesized by the re-screened Enz.24 in Table 10.
Figure 7 presents the synthetic route for preparing rebaudioside A and rebaudioside I from stevioside.
Figure 8 presents the chromatogram of the reference substance of rebaudioside A using the HPLC detection method in examples 8-11.
Figure 9 presents the chromatogram of the reference substance of rebaudioside I using the HPLC detection method in examples 8-11.
Figure 10 is an HPLC chromatogram of the reaction for 8 h in example 11.
Figure 11 is an HPLC chromatogram of the reaction for 24 h in example 11.

### DETAILED DESCRIPTION OF THE PREFERRED EMBODIMENT

The present disclosure is further illustrated by way of examples, but it is not limited to the scope of these examples. Experimental methods that do not indicate specific conditions in the following examples were selected according to conventional methods and conditions, or according to the product's instruction manual.

Unless otherwise specified, the experimental methods used in the present disclosure are conventional methods. For specific gene cloning operations, please refer to the "Molecular Cloning: A Laboratory Manual" by J. Sambrook *et al.*

Unless otherwise specified, the abbreviations for amino acids used in the present disclosure are conventional in the field. The specific abbreviated symbols corresponding to the amino acids are shown in Table 1.

**Table 1**

| Names of amino acids | Three-letter symbols | One-letter symbols | Names of amino acids | Three-letter symbols | One-letter symbols |
|---|---|---|---|---|---|
| Alanine | Ala | A | Leucine | Leu | L |
| Arginine | Arg | R | Lysine | Lys | K |
| Asparagine | Asn | N | Methionine | Met | M |
| Aspartic acid | Asp | D | Phenylalanine | Phe | F |
| Cysteine | Cys | C | Proline | Pro | P |
| Glutanine | Gln | Q | Serine | Ser | S |
| Glutamic acid | Glu | E | Threonine | Thr | T |
| Glicine | Gly | G | Tryptophan | Trp | W |
| Histidine | His | H | Tyrosine | Tyr | Y |
| Isoleucine | Ile | I | Valine | Val | V |

The codons corresponding to the amino acids are also conventional in the field. The specific correspondence between amino acids and codons is shown in Table 2.

**Table 2**

| First nucleotide | Second nucleotide | | | | Third nucleotide |
|---|---|---|---|---|---|
| | T | C | A | G | |
| T | Phenylalanine (F) | Serine (S) | Tyrosine (Y) | Cysteine (C) | T |
| | Phenylalanine (F) | Serine (S) | Tyrosine (Y) | Cysteine (C) | C |
| | Leucine (L) | Serine (S) | Stop codon | Stop codon | A |
| | Leucine (L) | Serine (S) | Stop codon | Tryptophan (W) | G |
| C | Leucine (L) | Proline (P) | Histidine (H) | Arginine (R) | T |
| | Leucine (L) | Proline (P) | Histidine (H) | Arginine (R) | C |
| | Leucine (L) | Proline (P) | Glutamine (Q) | Arginine (R) | A |
| | Leucine (L) | Proline (P) | Glutamine (Q) | Arginine (R) | G |
| A | Isoleucine (I) | Threonine (T) | Asparagine (N) | Serine (S) | T |
| | Isoleucine (I) | Threonine (T) | Asparagine (N) | Serine (S) | C |
| | Isoleucine (I) | Threonine (T) | Lysine (K) | Arginine (R) | A |
| | Methionine (M) | Threonine (T) | Lysine (K) | Arginine (R) | G |
| G | Valine (V) | Alanine (A) | Aspartic acid (D) | Glycine (G) | T |
| | Valine (V) | Alanine (A) | Aspartic acid (D) | Glycine (G) | C |
| | Valine (V) | Alanine (A) | Glutamic acid (E) | Glycine (G) | A |
| | Valine (V) | Alanine (A) | Glutamic acid (E) | Glycine (G) | G |

KOD Mix enzyme was purchased from TOYOBO CO., LTD. *DpnI* enzyme was purchased from Invitrogen (Shanghai) Trading Co., Ltd. *E. coli* Trans 10 competent cells and *E.coli* BL21 (DE3) competent cells were both purchased from Beijing Dingguo Changsheng Biotechnology Co., Ltd. Sucrose was purchased from Sangon Biotech (Shanghai) Co., Ltd. The reaction substrate used in the first round of screening in examples 1-7 and RA60 (stevioside with RA content of 60% and stevioside content of about 30%) used in examples 8-11 were purchased from Chenguang Biotech, with the product specification TSG90/RA60. The reaction substrate stevioside used in the second round of screening was purchased from Bide Pharm (with a purity of 95%). The reference substances Reb A and Reb I were purchased from Macklin.

HPLC detection method for conversion rate in examples 1-7: chromatographic column: Agilent 5 TC-C18 (2) (250×4.6 mm). Mobile Phases: 0.1% FA aqueous solution as mobile phase A and 0.1% FA-acetonitrile solution as mobile phase B. Gradient elution was performed according to Table 3. Detection wavelength: 210 nm; flow rate: 1 mL/min; injection volume: 20 µL; column temperature: 40°C. Peak time of stevioside: 12.76 min; peak time of Reb A: 12.38 min.

**Table 3**

| **Time (min)** | **Mobile phase A%** | **Mobile phase B%** |
|---|---|---|
| 0.00 | 70 | 30 |
| 15.00 | 60 | 40 |
| 20.00 | 30 | 70 |
| 25.00 | 30 | 70 |
| 25.10 | 70 | 30 |
| 32.00 | 70 | 30 |

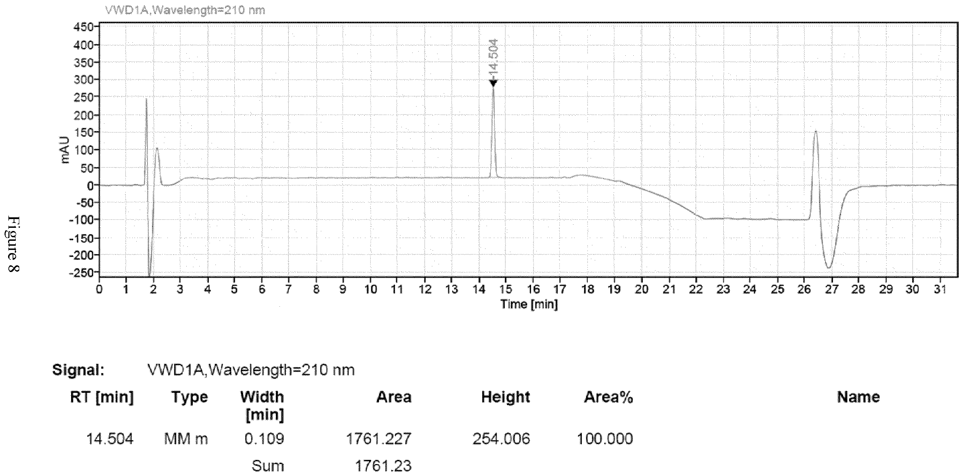

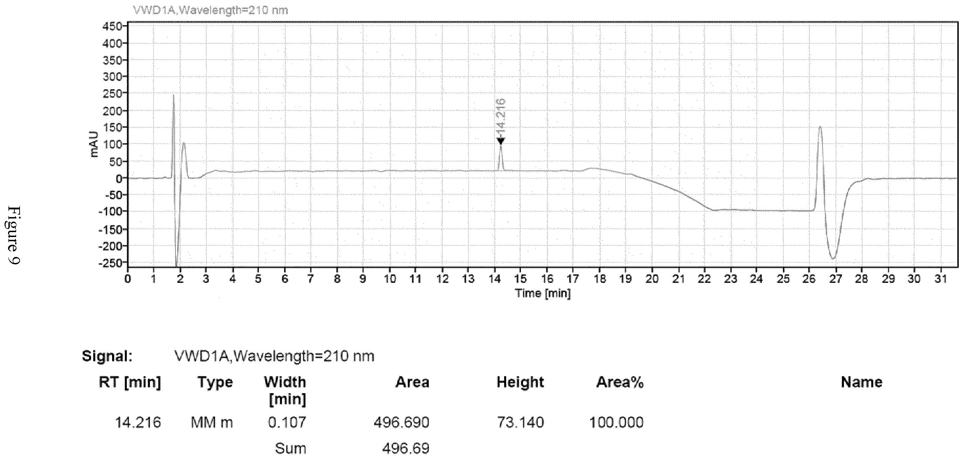

HPLC detection method for conversion rate in examples 8-11: chromatographic column: ZORBAXEclipse plus C18 (4.6 mm×150 mm, 3.5 µm). Mobile Phases: 0.1% TFA aqueous solution as mobile phase A and 0.1% TFA-acetonitrile solution as mobile phase B. Gradient elution was performed according to Table 4. Detection wavelength: 210 nm; flow rate: 1 mL/min; injection volume: 20 µl; column temperature: 35°C. As shown in Figure 8, peak time of Reb A: 14.504 min; as shown in Figure 9, peak Time for Reb I: 14.216 min.

**Table 4**

| Time (min) | A% | B% |
|---|---|---|
| 0.00 | 90 | 10 |
| 15.00 | 60 | 40 |
| 20.00 | 0 | 100 |
| 24.00 | 0 | 100 |
| 24.10 | 90 | 10 |
| 32.00 | 90 | 10 |

In examples 1 to 7, the parent of UDP-glucosyltransferase Enz.1 was primarily used as a template. In the first round of screening, a glycosyltransferase mutant, Enz.11 with a mutation of N at position 308 was screened out. Using Enz.11 as a template in the second round of screening, UDP-glucosyltransferase with double or triple site mutations was screened out.

### Example 1: Construction of the GT010-308 mutant library in the first round

The β-1,3-glycosyltransferase (β-1,3-GT enzyme) enzyme gene numbered Enz.1 shown in SEQ ID NO: 1 was fully synthesized. This gene was linked to the pET28a plasmid vector to obtain the recombinant plasmid pET28a-GT010. The gene synthesis was performed by Sangon Biotech Co., Ltd., located at No. 698 Xiangmin Road, Songjiang District, Shanghai. The amino acid sequence of Enz. 1 is as shown in SEQ ID NO: 2.

The pET28a-GT010 plasmid was used as a template, and the primer sequences shown in Table 5 were used. The primer pairs used were GT010-L308X-F/ET-R and ET-F/GT010-L308X-R, respectively (where X represents: A, D, E, G, H, I, K, M, N, P, S, V, W). PCR amplification was conducted by using KOD enzyme to amplify the target DNA and vector fragments.

**Table 5**

| Primer name | Primer sequence | SEQ ID NO: |
|---|---|---|
| GT010-L308A-F | GCTTTCTGTGGGCGGTTCGTCCGGGCTTCGTG | 3 |
| GT010-L308A-R | GGACGAACCGCCCACAGAAAGCTTTGTTTG | 4 |
| GT010-L308D-F | GCTTTCTGTGGGATGTTCGTCCGGGCTTCGTG | 5 |
| GT010-L308D-R | GGACGAACATCCCACAGAAAGCTTTGTTTG | 6 |
| GT010-L308E-F | GCTTTCTGTGGGAGGTTCGTCCGGGCTTCGTG | 7 |
| GT010-L308E-R | GGACGAACCTCCCACAGAAAGCTTTGTTTG | 8 |
| GT010-L308G-F | GCTTTCTGTGGGGTGTTCGTCCGGGCTTCGTG | 9 |
| GT010-L308G-R | GGACGAACACCCCACAGAAAGCTTTGTTTG | 10 |
| GT010-L308H-F | GCTTTCTGTGGCATGTTCGTCCGGGCTTCGTG | 11 |
| GT010-L308H-R | GGACGAACATCCCACAGAAAGCTTTGTTTG | 12 |
| GT010-L308I-F | GCTTTCTGTGGATTGTTCGTCCGGGCTTCGTG | 13 |
| GT010-L308I-R | GGACGAACAATCCACAGAAAGCTTTGTTTG | 14 |
| GT010-L308K-F | GCTTTCTGTGGAAGGTTCGTCCGGGCTTCGTG | 15 |
| GT010-L308K-R | GGACGAACCTTCCACAGAAAGCTTTGTTTG | 16 |
| GT010-L308M-F | GCTTTCTGTGGATGGTTCGTCCGGGCTTCGTG | 17 |
| GT010-L308M-R | GGACGAACCATCCACAGAAAGCTTTGTTTG | 18 |
| GT010-L308N-F | GCTTTCTGTGGAACGTTCGTCCGGGCTTCGTG | 19 |
| GT010-L308N-R | GGACGAACGTTCCACAGAAAGCTTTGTTTG | 20 |
| GT010-L308P-F | GCTTTCTGTGGCCGGTTCGTCCGGGCTTCGTG | 21 |
| GT010-L308P-R | GGACGAACCGGCCACAGAAAGCTTTGTTTG | 22 |
| GT010-L308S-F | GCTTTCTGTGGAGCGTTCGTCCGGGCTTCGTG | 23 |
| GT010-L308S-R | GGACGAACGCTCCACAGAAAGCTTTGTTTG | 24 |
| GT010-L308V-F | GCTTTCTGTGGGTGGTTCGTCCGGGCTTCGTG | 25 |
| GT010-L308V-R | GGACGAACCACCCACAGAAAGCTTTGTTTG | 26 |
| GT010-L308W-F | GCTTTCTGTGGTGGGTTCGTCCGGGCTTCGTG | 27 |
| GT010-L308W-R | GGACGAACCCACCACAGAAAGCTTTGTTTG | 28 |
| ET-F | CTTGTCTGCTCCCGGCATC | 29 |
| ET-R | CTTGTCTGTAAGCGGATGCC | 30 |

The PCR amplification reaction system is:

| Reagent | Volume (µL) |
|---|---|
| KOD Mix enzyme | 25 |
| Primer F | 2 |
| Primer R | 2 |
| Template | 1 |
| Deionized water | 20 |

The amplification program is as follows:

| | | |
|---|---|---|
| 98°C | 5 min | |
| 98°C | 10s | 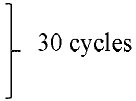 |
| 55°C | 5 s | |
| 68°C | 5 min | |
| 68°C | 10 min | |
| 12°C | ∞ | |

The PCR products were digested with *Dpn*I enzyme and then subjected to gel electrophoresis and gel recovery to obtain the target DNA fragments. These fragments were connected by using a two-fragment homologous recombinase (Exnase II) from Novagen. After ligation, the ligation mixture was transformed into *E. coli* Trans10 competent cells. These cells were then spread onto LB agar plates containing 50 µg/mL kanamycin and incubated overnight at 37°C. Colonies were picked from the plate for further culture and sequencing to obtain the recombinant plasmid containing the mutant gene.

### Example 2: Preparation of UDP-glucosyltransferase mutant

### 1. Protein expression of the mutant vector:

The above recombinant plasmid with correct sequencing was transformed into host *E.coli* BL21 (DE3) competent cells to obtain a genetically engineered strain containing point mutations. A single colony was picked and inoculated into 5 mL of LB liquid medium containing 50 µg/mL kanamycin and then incubated at 37°C for 4 h with shaking. The inoculum was transferred to 50 mL of fresh TB liquid medium containing 50 µg/mL kanamycin at a 2 v/v % inoculation rate. The culture was shaken at 37°C until the OD₆₀₀ reaches 0.6 to 0.8. IPTG (Isopropyl β-D-thiogalactoside) was then added to a final concentration of 0.1 mM, and the culture was induced at 25°C for 20 h. After cultivation, the culture was centrifuged at 10,000 rpm for 10 min, the supernatant was discarded, and the bacterial cells (i.e., bacterial sludge) were collected and stored at -20°C for later use.

### 2. Acquisition of the crude enzyme solution:

A phosphate buffer solution (PBS) of 50 mM with pH 6.0 was prepared. The bacterial sludge obtained above was suspended in this buffer at a ratio of 1:5 (M/V). This suspension was homogenized to obtain a crude enzyme solution. Subsequently, the crude enzyme solution was centrifuged and the supernatant was taken to obtain the crude enzyme solution of the UDP-glucosyltransferase mutant.

### Example 3: Preparation of sucrose synthase (SUS)

The sucrose synthase (SUS) gene numbered Enz.2 shown in SEQ ID NO: 31 was fully synthesized. This gene was linked to the pET28a plasmid vector to obtain the recombinant plasmid pET28a-SUS. The gene synthesis was performed by Sangon Biotech Co., Ltd., located at No. 698 Xiangmin Road, Songjiang District, Shanghai. The amino acid sequence of the sucrose synthase is as shown in SEQ ID NO: 32.

The plasmid pET28a-SUS was transformed into the host *E.coli* BL21 (DE3) competent cells to obtain the Enz.2 genetically engineered strain. A single colony was picked and inoculated into 5 mL of LB liquid medium containing 50 µg/mL kanamycin and then incubated at 37°C for 4 h with shaking. The inoculum was transferred to 50 mL of fresh TB liquid medium containing 50 µg/mL kanamycin at a 2 v/v % inoculation rate. The culture was shaken at 37°C until the OD₆₀₀ reaches 0.6 to 0.8. IPTG was then added to a final concentration of 0.1 mM, and the culture was induced at 25°C for 20 h. After cultivation, the culture was centrifuged at 10,000 rpm for 10 min (for use in examples 4-7) or at 4,000 rpm for 20 min (for use in examples 8-11). The supernatant was discarded, and the bacterial cells were collected and stored at -20°C for later use.

For examples 4-7: 50 mM phosphate buffer solution (PBS) with pH 6.0 was prepared. The bacterial sludge of Enz.2 obtained above was suspended at a ratio of 1:5 (M/V). The suspension was homogenized to obtain a crude enzyme solution. Subsequently, the crude enzyme solution was centrifuged, and the supernatant was taken to obtain the crude enzyme solution of sucrose synthase (enzyme No. Enz.2, with amino acid sequence as shown in SEQ ID NO: 32).

For examples 8-11: 50 mM phosphate buffer solution (PBS) with pH 6.0 was prepared. The bacterial sludge obtained above was suspended at a ratio of 1:10 (M/V, g/mL) and then homogenized under high pressure (550 Mbar, 1.5 min) and centrifuged at 12,000 rpm for 2 min to obtain sucrose synthase reaction enzyme solution.

### Example 4: Screening of mutants in the first round

The crude enzyme solutions obtained in example 2 and example 3 were incubated at a constant temperature of 80°C for 20 min, respectively. After centrifugation, the supernatants were collected to obtain UDP-glucosyltransferase mutant reaction enzyme solution and sucrose synthase reaction enzyme solution, respectively.

Using RA60 as the substrate, in a 1 mL reaction system, 150 µL of the UDP-glucosyltransferase mutant reaction enzyme solution was added, with a final concentration of RA60 of 100 g/L, UDP of 0.1 g/L, sucrose of 200 g/L, and 30 µL of the sucrose synthase reaction enzyme solution. Finally, 50 mM phosphate buffer with pH 6.0 was added to a final volume of 1 mL. The prepared reaction system was placed in a metal bath at 60°C and 600 rpm for 60 min. The reaction mixture was then diluted 50 times and subjected to HPLC to analyze the concentration of Reb A (see Table 6 for details of Reb A%). The sucrose synthase was used to transfer the glucose group from sucrose to UDP to synthesize UDP-glucose (UDPG). The initial screening results are shown in Table 6.

**Table 6**

| Enzyme No. | Mutation site | Reb A% | DNA SEQ ID NO: |
|---|---|---|---|
| Enz.1 | / | 93.787 | 2 |
| Enz.3 | L308A | 87.771% | 33 |
| Enz.4 | L308D | 72.570% | 34 |
| Enz.5 | L308E | 74.487% | 35 |
| Enz.6 | L308G | 81.027% | 36 |
| Enz.7 | L308H | 89.885% | 37 |
| Enz.8 | L308I | 83.863% | 38 |
| Enz.9 | L308K | 75.395% | 39 |
| Enz. 10 | L308M | 84.221% | 40 |
| Enz. 11 | L308N | 96.157% | 41 |
| Enz. 12 | L308P | 75.643% | 42 |
| Enz. 13 | L308S | 72.799% | 43 |
| Enz. 14 | L308V | 87.985% | 44 |
| Enz. 15 | L308W | 91.806% | 45 |

From the initial screening results in Table 6, it is observed that compared to Enz. 1, Enz. 11 demonstrates better catalytic effect, and the yield of Reb A is higher.

### 2. Re-screening

Re-screening was conducted under two reaction conditions: Boil and UnBoil. The Boil re-screening conditions are the same as those of the initial screening. The UnBoil conditions refers to the reaction without heating, while all other conditions are the same as those of the Boil reaction. The results of the re-screening are shown in Table 7 (the percentages in the table correspond to the content of Reb A in the reaction solution).

**Table 7**

| Enzyme No. | Enz.3 | Enz.7 | Enz.1 | Enz.11 | Enz.14 | Enz.15 |
|---|---|---|---|---|---|---|
| Boil | 84.212% | 85.939% | 92.314% | 92.100% | 83.793% | 87.168% |
| UnBoil | 79.577% | 82.266% | 84.522% | 83.475% | 80.686% | 81.180% |

From the re-screening results shown in Table 7, it can be seen that compared to Enz. 1, Enz. 11 has the best effect, and its reaction effect is comparable to that of Enz. 1.

### Example 5: Construction of the mutant library in the second round

The gene encoding the glycosyltransferase (β-1,3-GT enzyme) Enz. 11, obtained in the first round, was linked to the pET28a vector to obtain the recombinant plasmid pET28a-Enz. 11. Using pET28a-Enz. 11 as a template and the primer sequences shown in Table 8, KOD enzyme was used for PCR amplification to obtain gene fragments and vector fragments of the target mutants Enz. 16 to Enz. 3 4.

Using plasmid pET28a-Enz.34 as a template, GT029-L378G-F/Km-R and Km-F/GT029-L378G-R as primer sequences, gene fragments and vector fragments of the target mutant Enz. 35 were amplified by PCR.

**Table 8**

| Enzyme No. | Primer name | Sequence | SEQ ID NO: |
|---|---|---|---|
| Enz. 16 | GT029-R12Q-F | GCGTCGTCAGCGTGTTATTATGTTTCCGG | 46 |
| | GT029-R12Q-R | CATAATAACACGCTGACGACGCACGGTAGTTGG | 47 |
| Enz. 17 | GT029-V14I-F | CGTCGTATCATTATGTTTCCGGTTCCGTTC | 48 |
| | GT029-V14I-R | GAAACATAATGATACGACGACGACGCACGGTAG | 49 |
| Enz. 18 | GT029-E99L-F | CGCGGATCTATTCCGTAAGGAGCTGGAAATC | 50 |
| | GT029-E99L-R | CCTTACGGAATAGATCCGCGCCGTGTTTGTTA | 51 |
| Enz.19 | GT029-A118S-F | GGAAGTTAGTTGCGTGATTACCGATGCGC | 52 |
| | GT029-A118S-R | GTAATCACGCAACTAACTTCCTCGTCACTCGGA G | 53 |
| Enz.20 | GT029-S194P-F | CGCGCGTATCCGCACATTCAAGAATCGAAAC | 54 |
| | GT029-S194P-R | CTTGAATGTGCGGATACGCGCGACGGATATC | 55 |
| Enz.21 | GT029-T254G-F | CTTCGAGCGGTAGCCTGCTGGATACTGATCC | 56 |
| | GT029-T254G-R | CAGCAGGCTACCGCTCGAAGCCCTATAATGC | 57 |
| Enz.22 | GT029-L257A-F | GCACTAGCCTGGCGGATACTGATCCGAGCACCG | 58 |
| | GT029-L257A-R | CGGATCAGTATCCGCCAGGCTAGTGCTCGAAGC C | 59 |
| Enz.23 | GT029-E418D-F | GTTGACCCTGACGGTGAGGTTATGCGCCAAAAC | 60 |
| | GT029-E418D-R | CCTCACCGTCAGGGTCAACCATCACAC | 61 |
| Enz.24 | GT029-Q451E-F | GAACGCCTGGAGAGCTATATTAGCAGCCTG | 62 |
| | GT029-Q451E-R | CTAATATAGCTCTCCAGGCGTTCCAGGCTCTCG | 63 |
| Enz.25 | GT029-S455R-F | GAGCTATATTCGCAGCCTGTAAGCTTGCGG | 64 |
| | GT029-S455R-R | CTTACAGGCTGCGAATATAGCTCTGCAGGCGTTC | 65 |
| Enz.26 | GT029-Q265E-F | CCGCCGAATGGCTGGACCAGCAGCCGCC | 66 |
| | GT029-Q265E-R | GTCCAGCCATTCGGCGGTGCTCGGATCAGTATC | 67 |
| Enz.27 | GT029-P271A-F | GACCAGCAGGCGCCGAGCAGCGTGCTGTACG | 68 |
| | GT029-P271A-R | CTGCTCGGCGCCTGCTGGTCCAGCCATTGGG | 69 |
| Enz.28 | GT029-R333K-F | GAAAAAGGTAAGATCGTGAAGTCTGCTCCG | 70 |
| | GT029-R333K-R | CTTCACGATCTTACCTTTTTCACCCAGAAAAC | 71 |
| Enz.29 | GT029-K347P-F | CTGGCGCACCCTGCGATTGGTGCGTTCTGGAC | 72 |
| | GT029-K347P-R | ACCAATCGCAGGGTGCGCCAGCACTTCTTG | 73 |
| Enz.30 | GT029-L378G-F | GATTTCGGTGGTGATCAGCCGCTGAACGCGCG | 74 |
| | GT029-L378G-R | CGGCTGATCACCACCGAAATCGCTAAAGATC | 75 |
| Enz.31 | GT029-Q265E-P271A-F | CGAATGGCTGGACCAGCAGGCGCCGAGCAGCG TGCTGTAC | 76 |
| | GT029-Q265E-P271A-R | GCGCCTGCTGGTCCAGCCATTCGGCGGTGCTCG GATCAGTAT | 77 |
| Enz.32 | GT029-R333K-K347P-F | AAGATCGTGAAGTCTGCTCCGCAACAAGAAGTG CTGGCGCACCCTGCGATTGGTGCGTTCTGGAC | 78 |
| | GT029-R333K-K347P-R | AGGGTGCGCCAGCACTTCTTGTTGCGGAGCAGA CTTCACGATCTTACCTTTTTCACCCAG | 79 |
| Enz.33 | GT029-Q265E-P271A-F | CGAATGGCTGGACCAGCAGGCGCCGAGCAGCG TGCTGTAC | 80 |
| | GT029-L378G-R | CGGCTGATCACCACCGAAATCGCTAAAGATC | 81 |
| | GT029-L378G-F | GATTTCGGTGGTGATCAGCCGCTGAACGCGCG | 82 |
| | GT029-Q265E-P271A-R | GCGCCTGCTGGTCCAGCCATTCGGCGGTGCTCG GATCAGTAT | 83 |
| Enz.34 | GT029-R333K-K347P-F | AAGATCGTGAAGTCTGCTCCGCAACAAGAAGTG CTGGCGCACCCTGCGATTGGTGCGTTCTGGAC | 84 |
| | GT029-Q265E-P271A-R | GCGCCTGCTGGTCCAGCCATTCGGCGGTGCTCG GATCAGTAT | 85 |
| | GT029-Q265E-P271A-F | CGAATGGCTGGACCAGCAGGCGCCGAGCAGCG TGCTGTAC | 86 |
| | GT029-R333K-K347P-R | AGGGTGCGCCAGCACTTCTTGTTGCGGAGCAGA CTTCACGATCTTACCTTTTTCACCCAG | 87 |
| Enz. 3 5 | GT029-L378G-F | GATTTCGGTGGTGATCAGCCGCTGAACGCGCG | 88 |
| | GT029-L378G-R | CGGCTGATCACCACCGAAATCGCTAAAGATC | 89 |
| / | Km-F | GCCCGACATTATCGCGAGC | 90 |
| | Km-R | GGGTATAAATGGGCTCGCG | 91 |

The PCR amplification reaction system is shown in Table 9-1:

**Table 9-1**

| Reagent | Volume (µL) |
|---|---|
| KOD Mix | 25 |
| Primer F | 2 |
| Primer R | 2 |
| Template | 1 |
| Deionized water | 20 |

The amplification program is shown in Table 9-2:

**Table 9-2**

| | | |
|---|---|---|
| 98°C | 3min | |
| 98°C | 10s | 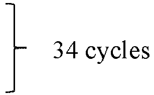 |
| 55°C | 5s | |
| 68°C | 5 s/kbp | |
| 68°C | 10min | |
| 12°C | ∞ | |

The PCR products were digested with *Dpn*I enzyme and then subjected to gel electrophoresis and gel recovery. These fragments were connected using a two-fragment homologous recombinase from Novagen. After ligation, the ligation mixture was transformed into *E.coli* Trans10 competent cells. These cells were then spread onto LB agar plates containing 50 µg/mL kanamycin and incubated overnight at 37°C. Colonies were picked from the plate for further culture and sequencing to obtain the recombinant plasmid containing the mutant gene.

### Example 6: Preparation of UDP-glucosyltransferase mutant

### 1. Protein expression of the mutant vector:

The recombinant plasmid correctly sequenced in example 5 was transformed into the host *E.coli* BL21 (DE3) competent cells to obtain a genetically engineered strain containing point mutations. A single colony was picked and inoculated into 5 mL of LB liquid medium containing 50 µg/mL kanamycin and then incubated at 37°C for 4 h with shaking. The inoculum was transferred to 50 mL of fresh TB liquid medium containing 50 µg/mL kanamycin at a 2 v/v % inoculation rate. The culture was shaken at 37°C until the OD₆₀₀ reaches 0.6 to 0.8. IPTG was then added to a final concentration of 0.1 mM, and the culture was induced at 25°C for 20 h. After cultivation, the culture was centrifuged at 4,000 rpm for 20 min, the supernatant was discarded, and the bacterial cells (i.e., bacterial sludge) were collected and stored at -20°C for later use.

### 2. Acquisition of the crude enzyme solution:

A phosphate buffer solution (PBS) of 50 mM with pH 6.0 was prepared. The bacterial sludge obtained above was suspended in this buffer at a ratio of 1:10 (M/V). The suspension was homogenized to obtain a crude enzyme solution. Subsequently, the crude enzyme solution was centrifuged and the supernatant was taken to obtain the crude enzyme solution of the UDP-glucosyltransferase mutant. The crude enzyme solution was stored at - 4°C for later use.

### Example 7: Screening of mutants in the second round

### 1. Initial screening

The crude enzyme solutions obtained in examples 6 and 2 were incubated at a constant temperature of 80°C for 15 min, respectively. After centrifugation, the supernatants were collected to obtain the UDP-glucosyltransferase reaction enzyme solution and the sucrose synthase reaction enzyme solution, respectively.

Using stevioside (with a stevioside content of 95%, Bide Pharm) as the substrate, in a 1 mL reaction system 150 µL of the UDP-glucosyltransferase mutant reaction enzyme solution was added, with a final concentration of stevioside of 100 g/L, UDP of 0.1 g/L, sucrose of 200 g/L, and 30 µL of the sucrose synthase. Finally, 50 mM phosphate buffer with pH 6.0 was added to a final volume of 1 mL. The prepared reaction system was placed in a metal bath at 60°C and 600 rpm for 60 min. The reaction mixture was then diluted 50 times and subjected to HPLC to analyze the concentration of Reb A. A total of 20 mutants were screened by using both Enz.1 and Enz. 11 as double controls. The initial screening results are shown in Table 10.

**Table 10**

| Enzyme No. | Mutation site | Reb A % | DNA SEQ ID NO: |
|---|---|---|---|
| Enz.1 | / | 46.679 | 1 |
| Enz.11 | L308N | 59.987 | 41 |
| Enz.16 | L308N/R12Q | 50.093 | 92 |
| Enz.17 | L308N/V14I | 72.071 | 93 |
| Enz.18 | L308N/E99L | 70.986 | 94 |
| Enz.19 | L308N/A118S | 44.798 | 95 |
| Enz.20 | L308N/S194P | 48.034 | 96 |
| Enz.21 | L308N/T254G | 68.596 | 97 |
| Enz.22 | L308N/L257A | 74.188 | 98 |
| Enz.23 | L308N/E418D | 46.268 | 99 |
| Enz.24 | L308N/Q451E | 76.599 | 100 |
| Enz.25 | L308N/S455R | 49.538 | 101 |
| Enz.26 | L308N/Q265E | 75.384 | 102 |
| Enz.27 | L308N/P271A | 70.993 | 103 |
| Enz.28 | L308N/R333K | 62.605 | 104 |
| Enz.29 | L308N/K347P | 22.588 | 105 |
| Enz.30 | L308N/L378G | 27.904 | 106 |
| Enz.31 | L308N/Q265E/ P271A | 64.389 | 107 |
| Enz.32 | L308N/R333K/ K347P | 65.756 | 108 |
| Enz.33 | L308N/Q265E/ P271A/ L378G | 32.417 | 109 |
| Enz.34 | L308N/Q265E/ P271A/ R333K/ K347P/ | 46.335 | 110 |
| Enz.35 | L308N/ Q265E/ P271A/ R333K/ K347P/ L378G | 32.366 | 111 |

In the table, the symbol "/" indicates the presence of mutations at different sites simultaneously.

From the results of the initial screening in Table 10, it can be seen that Enz.17, Enz.18, Enz.21, Enz.22, Enz.24, Enz.26, Enz.27, Enz.28, Enz.31, and Enz.32 are all superior to the control by more than 10%, and these mutants were selected for re-screening. Additionally, the aforementioned reaction involved incubating the crude enzyme solution at a constant temperature of 80°C for 15 min, centrifuging the supernatant to obtain the reaction enzyme solution, and then carrying out the reaction. It can be seen that the enzymes have good stability.

### 2. Re-screening

The reaction conditions for the re-screening are the same as those for the initial screening. The results of the re-screening are shown in Table 11.

**Table 11**

| Enzyme | Reb A % |
|---|---|
| Enz.1 | 47.066 |
| Enz.11 | 61.639 |
| Enz.17 | 73.639 |
| Enz.18 | 72.940 |
| Enz.21 | 66.063 |
| Enz.22 | 74.680 |
| Enz.24 | 76.903 |
| Enz.26 | 72.557 |
| Enz.27 | 63.514 |
| Enz.28 | 61.659 |
| Enz.31 | 66.672 |
| Enz.32 | 59.815 |

From the re-screening results in Table 11, it is confirmed that Enz.17, Enz.18, Enz.21, Enz.22, Enz.24, Enz.26, Enz.27, Enz.28, Enz.31, and Enz.32 are all superior to the parent control by more than 10%. Compared to the parent UDP-glucosyltransferase Enz.1, the UDP-glucosyltransferase mutants obtained above show a significant improvement in catalytic activity.

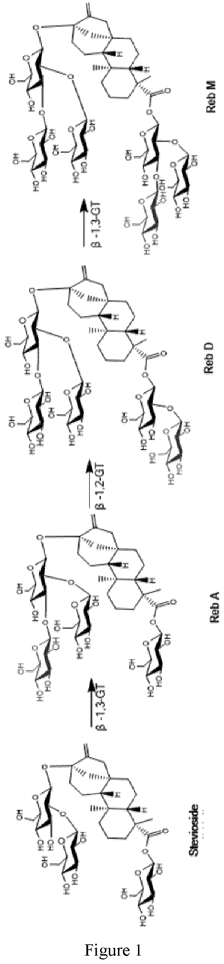

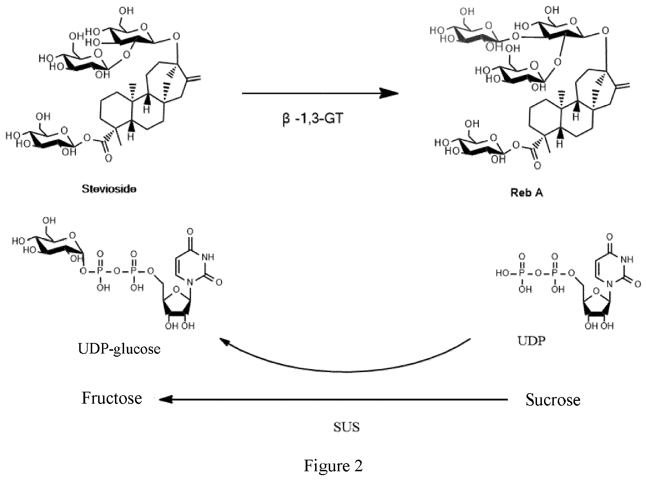

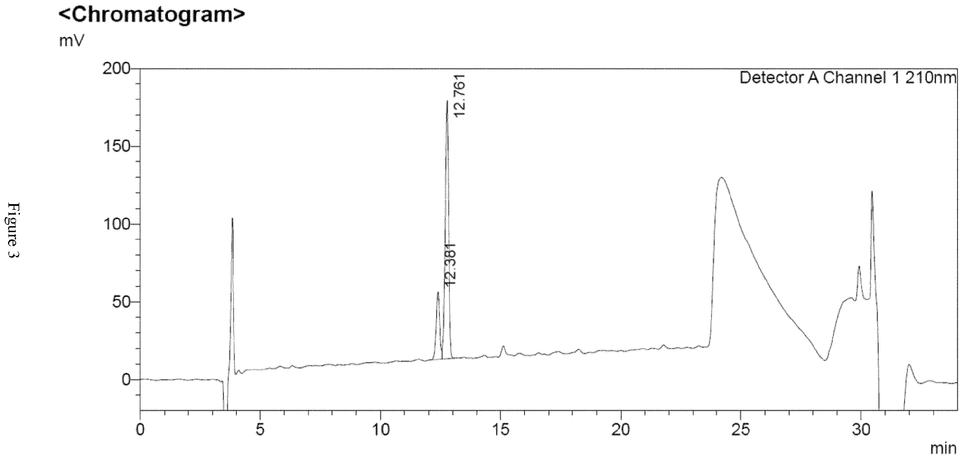

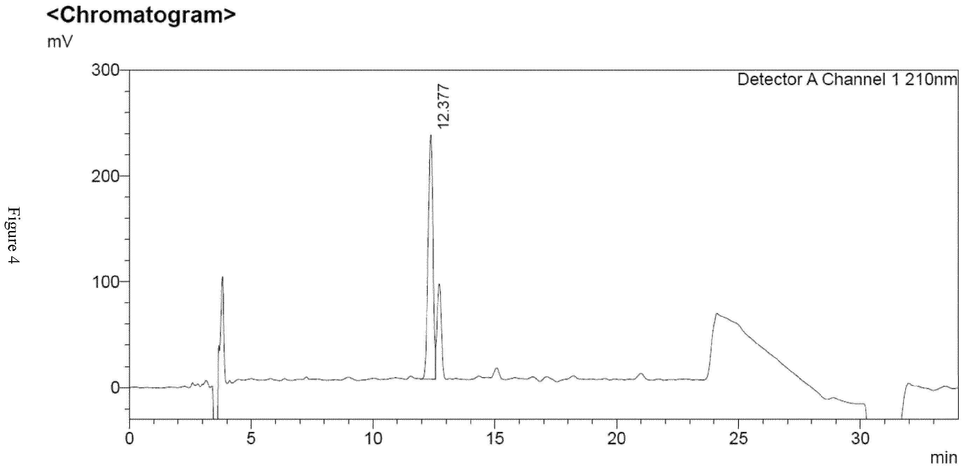

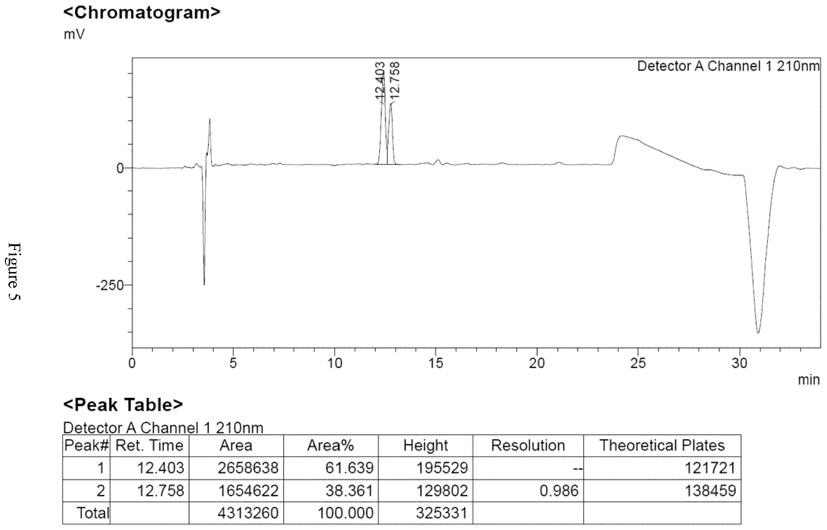

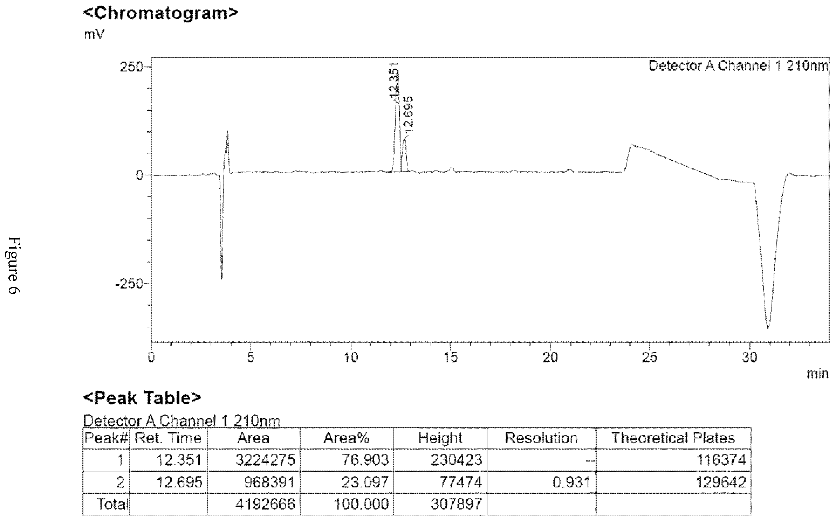

Figure 1 presents a schematic diagram of the route for preparing rebaudioside A, rebaudioside D, and rebaudioside M from stevioside in examples of the present disclosure. Figure 2 presents the enzyme-catalyzed reaction system in examples of the present disclosure for synthesizing rebaudioside A, demonstrating the cycling of UDPG in the biocatalytic reaction. Figure 3 presents a chromatogram of the substrate stevioside used in the second round of screening, with a retention time of 12.76 min. Figure 4 presents a chromatogram of the reference substance of the product rebaudioside A, with a retention time of 12.38 min. Figure 5 is the HPLC chromatogram of the activity of RA catalytic synthesized by the re-screened Enz.11 in Table 11. Figure 6 is the HPLC chromatogram of the activity of RA catalytic synthesized by the re-screened Enz.24 in Table 11.

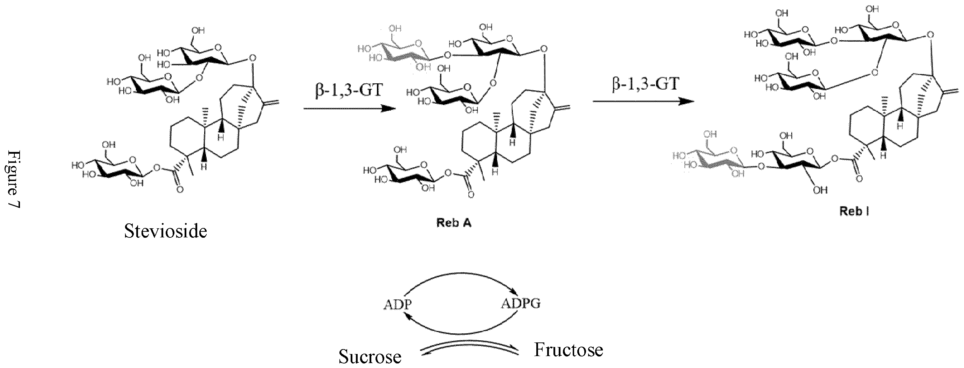

The schematic diagram of the route for examples 8-12 is illustrated in Figure 7.

### Example 8: Construction of β-1,3-glycosyltransferase mutant library in the first round

As in example 1, the fully synthesized β-1,3-(glycosyltransferase (β-1,3-GT enzyme) enzyme gene numbered Enz.1 as shown in SEQ ID NO: 1 was linked to the pET28a plasmid vector to obtain the recombinant plasmid pET28a-Enz.1. The gene synthesis was performed by Sangon Biotech Co., Ltd., located at No. 698 Xiangmin Road, Songjiang District, Shanghai. The amino acid sequence of Enz.1 is as shown in SEQ ID NO: 2. The recombinant plasmid was transformed into the host *E. coli* BL21 (DE3) competent cells to obtain an engineered strain containing the transaminase Enz.1 gene.

Similarly, the genes of the transaminases Enz. 2.2 to Enz. 2.6, and Enz. 16, Enz. 19, Enz. 23, Enz. 25, Enz. 27, Enz. 31, Enz. 32, and Enz. 35 were obtained by site-directed mutagenesis according to the Enz.1 gene in Table 12, with enzyme cleavage sites NdeI and HindIII, and ligation the vector pET28a to obtain recombinant plasmids containing the genes of the transaminases Enz. 2.2 to Enz. 2.6, and Enz. 16, Enz. 19, Enz. 23, Enz. 25, Enz. 27, Enz. 31, Enz. 32, and Enz. 35, respectively. Each recombinant plasmid was then transformed into the host *E.coli* BL21 (DE3) competent cells to obtain the engineered strains containing the transaminase genes as listed in Table 12.

**Table 12**

| Enzyme No. | Mutation sites (relative to Enz.1) | Codons of mutation sites (relative to Enz.1) |
|---|---|---|
| Enz.1 | / | / |
| Enz.2.2 | L308V / L257A / Q451E / I189L / G316S | 308GTG-257GCG-451GAG-189CTC-316AGT |
| Enz. 2.3 | L308V / L257A / Q451E / I189L / K347G | 308GTG-257GCG-451GAG-189CTC-347GGG |
| Enz. 2.4 | L308V / L257A / Q451E / I189L / P324G | 308GTG-257GCG-451GAG-189CTC-324GGG |
| Enz. 2.5 | L308V / L257A / Q451E / I189L / V14I | 308GTG-257GCG-451GAG-189CTC-14ATC |
| Enz. 2.6 | L308V / L257A / Q451E / I189L / S273G | 308GTG-257GCG-451GAG-189CTC-273 GGC |
| Enz. 16 | L308N / R12Q | 308AAC-12CAG |
| Enz. 19 | L308N/A118S | 308AAC-118TCT |
| Enz. 23 | L308N / E418D | 308AAC-418GAC |
| Enz. 25 | L308N / S455R | 308AAC-455CGT |
| Enz. 27 | L308N / P271A | 308AAC-271GCT |
| Enz. 31 | L308N / Q265E / P271A | 308AAC-265GAA-271GCT |
| Enz. 32 | L308N / R333K / K347P | 308AAC-333AAA-347CCG |
| Enz. 35 | L308N / Q265E / P271A / R333K / K347P / L378G | 308AAC-265GAA-271GCT-333AAA-347CCG-378GGT |

### Example 9: Preparation of β-1,3-glycosyltransferase mutant

### Protein expression of the mutant vector:

Single colonies of the strains in example 8 were picked and inoculated into 5 mL of LB liquid medium containing 50 µg/mL kanamycin and then incubated at 37°C for 4 h with shaking. The inoculum was transferred to 50 mL of fresh TB liquid medium containing 50 µg/mL kanamycin at a 2 v/v % inoculation rate. The culture was shaken at 37°C until the OD₆₀₀ reaches about 0.8. IPTG (Isopropyl β-D-thiogalactoside) was then added to a final concentration of 0.1 mM, and the culture was induced at 25°C for 20 h. After cultivation, the culture was centrifuged at 4,000 rpm for 20 min, the supernatant was discarded, and the bacterial cells were collected and stored at -20°C for later use.

### Example 10: Screening of β-1,3-glycosyltransferase mutants

### Acquisition of reaction enzyme solutions:

50 mM phosphate buffer solution (PBS) with pH 6.0 was prepared. The bacterial cells obtained in examples 6 and 9 were suspended at a ratio of 1:10 (M/V, g/mL). The suspension was then homogenized under high pressure by using a high-pressure homogenizer (550 Mbar for 1.5 min). The homogenized enzyme solution was centrifuged at 12,000 rpm for 2 min, respectively, to obtain the reaction enzyme solution for each β-1,3-glycosyltransferase.

In 1 mL reaction system,150 µL of the β-1,3-glycosyltransferase reaction enzyme solution was added, with a final concentration of RA60 of 50 g/L, ADP of 0.1 g/L, sucrose of 200 g/L, and 30 µL of the sucrose synthase reaction enzyme solution prepared in example 3. Finally, 50 mM phosphate buffer with pH 6.0 was added to a final volume of 1 mL. The prepared reaction system was placed in a metal bath and reacted at 60°C and 600 rpm for 30 min. Then, 10 µL of the reaction solution was added into 990 µL of hydrochloric acid (pH 2-3), vortexed, and centrifuged at 13,000 rpm for 10 min. The supernatant was subjected to HPLC to analyze the concentration of Reb I. Experimental results obtained using the HPLC detection method are shown in Table 13.

**Table 13**

| Enzyme No. | RI% (RA→RI) |
|---|---|
| Enz. 1 | 2.886 |
| Enz. 2.2 | 0.555 |
| Enz. 2.3 | 1.705 |
| Enz. 2.4 | 1.324 |
| Enz. 2.5 | 0.886 |
| Enz. 2.6 | 1.427 |
| Enz. 16 | 4.139 |
| Enz. 19 | 4.014 |
| Enz. 23 | 3.628 |
| Enz. 25 | 3.910 |
| Enz. 27 | 4.235 |
| Enz. 31 | 4.646 |
| Enz. 32 | 3.546 |
| Enz. 35 | 1.939 |

From the initial screening results in Table 13, it can be seen that Enz. 31 has the highest enzymatic activity. Therefore, Enz. 31 was selected for subsequent experiments.

### Example 11: Catalytic synthesis of RI by enzyme Enz. 31

In a 1 mL reaction system, 150 µL of the reaction enzyme solution of Enz. 31 was added, along with 30 µL of the sucrose synthase reaction enzyme solution, with a final concentration of RA60 of 100 g/L, sucrose of 200 g/L, and ADP of 0.1 g/L. Finally, PBS (50 mM, pH 5.5) was added to a final volume of 1 mL. The prepared reaction system was placed in a metal bath and reacted at 60°C and 600 rpm. Samples were taken at 1 h, 2 h, 3 h, 4 h, 5 h, 6 h, 7 h, 8 h, and 24 h, respectively. For each sample, 10 µL of reaction solution was added to 990 µL of hydrochloric acid (pH 2-3), vortexed, and centrifuged at 13,000 rpm for 10 min. The supernatant was analyzed by HPLC. Experimental results obtained using the HPLC detection method are shown in Table 14.

**Table 14**

| | | | | | | | | | |
|---|---|---|---|---|---|---|---|---|---|
| Time/h | 1 | 2 | 3 | 4 | 5 | 6 | 7 | 8 | 24 |
| RI% | 17.62 | 26.18 | 39.47 | 46.92 | 55.86 | 61.70 | 67.71 | 71.36 | 97.81 |

The reaction results in Table 14 show that after 8 h of reaction, the peak area ratio of RI reached 71%. After 24 h of reaction, the peak area ratio of RI reached 97%, indicating that the reaction was basically complete.

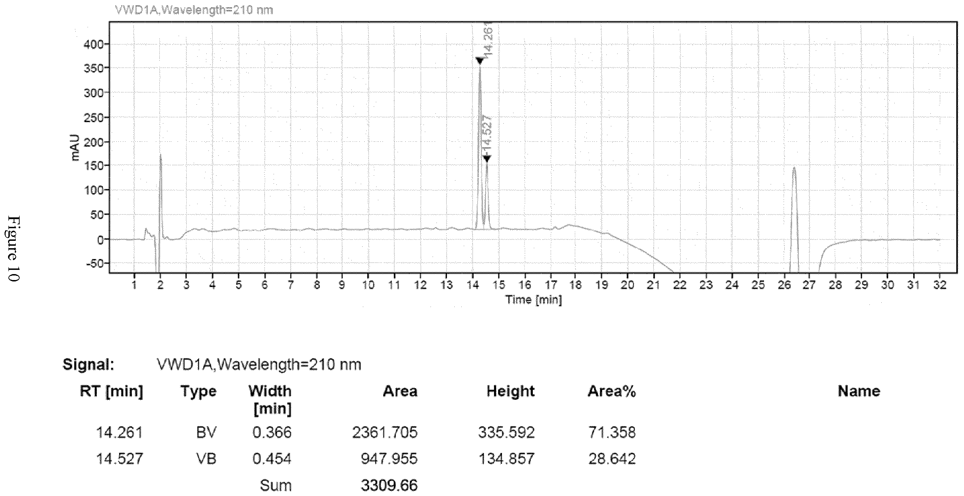

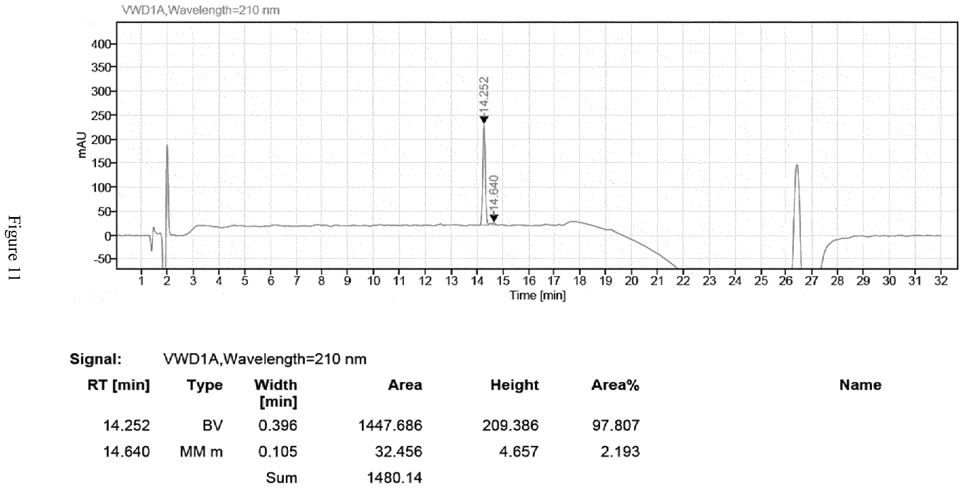

Figure 10 is the HPLC chromatogram of the experimental results of the reaction for 8 h in Table 14. Figure 11 is the HPLC chromatogram of the experimental results of the reaction for 24 h in Table 14.

Although specific embodiments of the present disclosure have been described above, it should be understood by those skilled in the field that these are merely illustrative examples. Various changes or modifications can be made to these embodiments without departing from the principles and essence of the present disclosure. Therefore, the scope of protection of the present disclosure is defined by the appended claims.

## Claims

1. A glycosyltransferase, wherein an amino acid sequence of the glycosyltransferaseis shown as SEQ ID NO: 112, or an amino acid sequence having at least 99% sequence identity with SEQ ID NO: 112.

2. The glycosyltransferase according to claim 1, wherein the amino acid sequence having at least 99% sequence identity with SEQ ID NO: 112 contains differences in amino acid residues selected from one or more of the following residue positions compared to SEQ ID NO: 112:
V14I;
E99L;
T254G;
L257A;
Q451E;
Q265E;
P271A;
R333K;
R12Q;
A118S;
E418D;
S455R;
preferably, the amino acid sequence of the glycosyltransferase also contains differences in amino acid residues selected from the following residue position compared to SEQ ID NO: 112: K347P.

3. The glycosyltransferase according to claim 2, wherein the amino acid sequence of the glycosyltransferase contains differences in amino acid residues selected from one of the following residue positions compared to SEQ ID NO: 112: V14I, E99L, T254G, L257A, Q451E, Q265E, P271A, R333K, R12Q, A118S, E418D, or S455R;
or, the amino acid sequence of the glycosyltransferase contains differences in amino acid residues selected from the following residue positions compared to SEQ ID NO: 112: Q265E and P271A;
or, the amino acid sequence of the glycosyltransferase contains differences in amino acid residues selected from the following residue positions compared to SEQ ID NO: 112: R333K and K347P.

4. The glycosyltransferase according to any one of claims 1 to 3, wherein a nucleotide sequence encoding the glycosyltransferase is selected from the following sequences: SEQ ID NO: 41, SEQ ID NO: 92, SEQ ID NO: 93, SEQ ID NO: 94, SEQ ID NO: 95, SEQ ID NO: 97, SEQ ID NO: 98, SEQ ID NO: 99, SEQ ID NO: 100, SEQ ID NO: 101, SEQ ID NO: 102, SEQ ID NO: 103, SEQ ID NO: 104, SEQ ID NO: 107, and SEQ ID NO: 108.

5. An isolated nucleic acid, wherein the nucleic acid encodes the glycosyltransferase according to any one of claims 1 to 4;
preferably, a nucleotide sequence of the nucleic acid is selected from the following sequences: SEQ ID NO: 41, SEQ ID NO: 92, SEQ ID NO: 93, SEQ ID NO: 94, SEQ ID NO: 95, SEQ ID NO: 97, SEQ ID NO: 98, SEQ ID NO: 99, SEQ ID NO: 100, SEQ ID NO: 101, SEQ ID NO: 102, SEQ ID NO: 103, SEQ ID NO: 104, SEQ ID NO: 107, SEQ ID NO: 108.

6. A recombinant expression vector comprising the nucleic acid according to claim 5.

7. A transformant comprising a host cell containing the nucleic acid according to claim 5 or the recombinant expression vector according to claim 6; preferably, the host cell is *Escherichia coli.*

8. A method for preparing the glycosyltransferase according to any one of claims 1 to 4, wherein the method includes culturing the transformant according to claim 7 under conditions suitable for expressing the glycosyltransferase.

9. A composition comprising the glycosyltransferase according to any one of claims 1 to 4.

10. A method for glycosylation a substrate, wherein the method includes providing at least one substrate and the glycosyltransferase according to any one of claims 1 to 4, and contacting the substrate with the glycosyltransferase under conditions that cause the substrate to be glycosylated to produce at least one glycosylation product.

11. A preparation method for rebaudioside A, wherein the preparation method includes the following steps: in the presence of the glycosyltransferase according to any one of claims 1 to 4, a reaction between stevioside and a glycosyl donor is carried out, thereby obtaining rebaudioside A;
preferably,
the glycosyltransferase exists in the form of a bacterial sludge;
and/or, a concentration of the stevioside is 1 to 150 g/L, preferably 100 g/L;
and/or, a molar ratio of the glycosyl donor to the stevioside is 1:1 to 5:1;
and/or, the glycosyl donor is UDP-glucose; it is preferably prepared by sucrose and UDP in the presence of sucrose synthase, and a concentration of the sucrose is preferably 100 to 300 g/L, for example, 200 g/L, and a concentration of the UDP is preferably 0.05 to 0.2 g/L, for example, 0.1 g/L;
and/or, a reaction solvent used in the reaction has a pH between 5 and 8, preferably 6;
and/or, a rotational speed during the reaction is 500 to 1000 rpm, preferably 600 rpm;
and/or, a temperature of a reaction system for the reaction is 20 to 90°C, preferably 60°C.

12. A preparation method for rebaudioside D or rebaudioside M, wherein the preparation method includes steps of preparing rebaudioside A according to the preparation method as claimed in claim 11.

13. A preparation method for rebaudioside I, reacting rebaudioside A with a glycosyl donor in the presence of a glycosyltransferase, wherein the glycosyltransferase is as claimed in any one of claims 1 to 4, and the preparation method satisfies one or more of the following conditions:
the glycosyltransferase exists in the form of glycosyltransferase cells, crude enzyme solution, pure enzyme, pure enzyme solution, or immobilized enzyme;
the glycosyl donor is UDP-glucose and/or ADP-glucose;
a concentration of the rebaudioside A is 1 to 150 g/L, preferably 100 g/L;
a mass ratio of the glycosyltransferase cells to rebaudioside A is 1 :(3-10), preferably 3:20;
a reaction solvent used in the reaction has a pH between 5 and 8, preferably 5.5 to 6;
a temperature of a reaction system for the reaction is 20 to 90°C, preferably 60°C;
a reaction time for the reaction is preferably 5 to 30 h, preferably 24 h.

14. The preparation method according to claim 13, wherein the glycosyl donor is prepared by UDP and/or ADP in the presence of sucrose and sucrose synthase;
and/or, a concentration of the sucrose is 100 to 300 g/L, preferably 200 g/L, and a concentration of the UDP is 0.05 to 0.2 g/L, preferably 0.1 g/L;
and/or, the pH is controlled by a buffer solution, and the buffer solution is preferably a phosphate buffer solution;
and/or, a rotation speed of the reaction is 500 to 1000 rpm, preferably 600 rpm.

15. A use of the glycosyltransferase according to any one of claims 1 to 4 in the preparation of steviol glycosides; the steviol glycosides are preferably rebaudioside A, rebaudioside D, rebaudioside M, or rebaudioside I.

16. A catalytic enzyme composition including a glycosyltransferase and a sucrose synthase, and the glycosyltransferase is as claimed in any one of claims 1 to 4, and the sequence of the sucrose synthase is as shown in SEQ ID NO: 32.

17. The catalytic enzyme composition according to claim 16, wherein a mass ratio of the glycosyltransferase to the sucrose synthase is preferably (2-10): 1, preferably 5:1.

18. A use of the catalytic enzyme composition according to claim 16 or 17 in the preparation of rebaudioside A, rebaudioside D, rebaudioside M, or rebaudioside I.
